# EUROPÄISCHE PATENTSCHRIFT

(11) **EP 2 075 034 B1**
(45) Veröffentlichungstag und Bekanntmachung des Hinweises auf die Patenterteilung: **18.11.2015**
(21) Anmeldenummer: 08172832.1
(22) Anmeldetag: 23.12.2008
(51) Int. Cl.: A61N 5/06

(54) **Bestrahlungssystem für Solarien**
Irradiation system for solariums
Système de rayonnement pour solarium

(30) Priorität: 29.12.2007 DE 102007063156
(43) Veröffentlichungstag der Anmeldung: 01.07.2009
(73) Patentinhaber: Vossloh-Schwabe Deutschland GmbH, 73660 Urbach (DE)
(72) Erfinder: Bulling, Martin, 73527 Schwäbisch Gmünd (DE); Raithel, Stefan, 73635 Rudersberg (DE); Braunschmid, Peter, 73430 Aalen (DE)
(74) Vertreter: Rüger, Barthelt & Abel

(56) Entgegenhaltungen:
- WO-A-98/46049
- DE-A1- 4 312 547
- DE-A1- 10 054 660
- DE-A1- 19 622 074
- DE-A1- 19 708 791
- DE-C1- 4 413 421

## Beschreibung

Die Erfindung betrifft eine Betriebseinrichtung für zumindest eine Gasentladungslampe einer Bestrahlungseinrichtung sowie die Bestrahlungseinrichtung.

Z.B. in Solarien werden zur kosmetischen Körperbräunung Bestrahlungseinrichtungen eingesetzt, die meist eine größere Anzahl von Leuchtstofflampen enthalten. Diese dienen dazu, den Nutzer mit UV-Licht zu bestrahlen.

Die Leuchtstofflampen sind meist speziell darauf eingerichtet, außer dem sichtbaren Licht relativ hohe UV-Anteile abzugeben. Die UV-Abgabe solcher Gasentladungslampen hängt vom Betriebsstrom und vom Alter der Gasentladungslampe ab und ist variabel.

Die Bestrahlung des Körpers mit UV-Licht hat häufig vorwiegend kosmetische Aspekte, kann ab er auch gesundheitsförderlich sein. Ebenso gehen aber mit der UV-Bestrahlung Gefahren einher. Solche Gefahren können sich insbesondere daraus ergeben, dass zu große oder zu kleine Strahlungsmengen auf den Körper einwirken. Bei herkömmlichen Solarien erfolgt die Exposition meist zeitgesteuert. Die Bestrahlungszeit kann aber, selbst wenn sie für die meisten Fälle angemessen ist, im Einzelfall zu einer zu großen oder zu niedrigen Dosis führen.

Aus DE 43 12 547 A1 sind eine Vorrichtung und ein Verfahren zur Bestrahlung des menschlichen Körpers bekannt. Im Bereich der Bestrahlungsröhren ist ein UV-Sensor zur Messung der aktuellen Strahlungsleistung angeordnet. Eine Steuereinrichtung prüft, ob die gemessene Strahlungsleistung den Vorgaben entspricht und regelt die Vorschaltgeräte der Bestrahlungsröhren erforderlichenfalls entsprechend. Gleichartige Vorrichtungen bzw. Verfahren ergeben sich auch aus WO 98/46049 sowie DE 100 54 660 A1.

DE 196 22 074 A1 betrifft eine Vorrichtung zur Vermessung von UV-Strahlung in Bräunungsgeräten und ein entsprechendes Messverfahren. Die Vorrichtung verfügt über einen Messkopf mit UV-Filtern und einem Diodenarray-Spektrometer sowie einen cosinuskorrigierten UVA-Messkopf mit Diffusor. Zunächst werden ein oder mehrere relative Spektren verschiedener Wellenlängenbereiche mittels des Diodenarray-Spektrometers erfasst. Anschließend wird der UVA-Messkopf mittels der gemessenen relativen Spektren kalibriert. Nachfolgend kann mit dem UVA-Messkopf eine ortsabhängige UVA-Intensitätsverteilung gemessen werden. Über einen PC wird dann ein ortsabhängiges absolutes Spektrum berechnet und mit der Erythemfunktion gewichtet.

Es ist die Aufgabe der Erfindung, eine Betriebseinrichtung zu schaffen, die die Sicherheit für den Benutzer erhöht.

Diese Aufgabe wird mit der Betriebseinrichtung nach Anspruch 1 wie auch mit der Bestrahlungseinrichtung gelöst, die eine solche Betriebseinrichtung enthält.

Die erfindungsgemäße Betriebseinrichtung enthält ein Beleuchtungssystem, das den abgegebenen UV-Lichtstrom unter Kontrolle bringt. Der UV-Lichtstrom wird mittels einer, vorzugsweise mehrerer Gasentladungslampen, beispielsweise Niederdruckgasentladungslampen mit Trägergas und Quecksilberfüllung und gegebenenfalls einem oder mehreren Leuchtstoffen erzeugt. Den Gasentladungslampen sind Vorschaltgeräte zugeordnet. Diese sind darauf eingerichtet, die Gasentladungslampen mit einem gewünschten Lampenstrom zu versorgen. Die Vorschaltgeräte können den Lampenstrom dabei in Grenzen variieren, um den Lichtstrom der angeschlossenen Gasentladungslampen zu erhöhen oder zu vermindern. Die Vorschaltgeräte sind mit einer Steuereinrichtung verbunden, die die gewünschten Lampenströme für die Vorschaltgeräte vorgibt. Die Steuereinrichtung ist wiederum mit zumindest einem, vorzugsweise aber mehreren Sensoreinrichtungen verbunden, die den UV-Lichtstrom an einer oder mehreren Stellen erfassen. Entsprechend den erfassten UV-Lichtströmen kann die Steuereinrichtung dann die Vorschaltgeräte mehr oder weniger dimmen, um den tatsächlichen UV-Lichtstrom mit dem gewünschten UV-Lichtstrom in Übereinstimmung zu bringen. Die hat Sensoreinrichtung eine Abhängigkeit der Ausgangssignalstärke von der Lichtwellenlänge, die zumindest für Lichtwellenlängen kleiner als eine Grenze mit dem Empfindlichkeitsprofil der menschlichen Haut in guter Näherung übereinstimmt.

Damit lassen sich insbesondere UV-Überdosierungen vermeiden wie sie sonst auftreten können. Beispielsweise liefern neue Leuchtstofflampen in ihren ersten Betriebsstunden einen sehr hohen UV-Lichtstrom, der dann auf einen niedrigeren Wert abfällt, der sich dann über mehrere hundert Stunden hinweg nur noch wenig ändert, um daraufhin wiederum stark abzufallen. Dieser Abfall des UV-Lichtstroms ist nicht unmittelbar mit dem sichtbaren Lichtstrom korreliert, so dass UV-Lampen mit zu hoher oder zu niedriger UV-Leistung häufig unentdeckt bleiben. Die individuelle oder auch gruppenweise Regulierung der Lampenströme anhand erfasster UV-Lichtströme ermöglicht es jedoch in einem solchen Solarium, den gewünschten UV-Lichtstrom einzuhalten und auch eine vom Nutzer aufgenommene UV-Dosis zu begrenzen und zwar auch dann, wenn aufgrund der Lampenalterung die UV-Lichtströme stark von der Norm abweichen.

Die erfindungsgemäße Betriebseinrichtung weist vorzugsweise eine Einrichtung zur Beeinflussung des durch die Gasentladungslampe fließenden Betriebsstroms auf. Diese Einrichtung kann beispielsweise die Schaltfrequenz einer in der Betriebseinrichtung vorhandenen Wechselrichtereinrichtung variieren. Sie kann alternativ andere Maßnahmen zur Beeinflussung des Lampenstroms verwirklichen, wie beispielsweise Änderung des Tastverhältnisses (Verhältnis von Einschaltzeit zu Ausschaltzeit) des Schaltsignals der Wechselrichtereinrichtung, einer Änderung der Gleichspannung, mit der die Wechselrichtereinrichtung betrieben wird, oder ähnliches.

Das Vorschaltgerät weist vorzugsweise eine Schnittstelle auf, über die der Lampenstrom vorgebbar ist. Diese Schnittstelle kann eine genormte Schnittstelle sein, die nach vorgegebenem Protokoll arbeitet. Als Vorschaltgerät kann ein elektronisches Vorschaltgerät mit Dimmschnittstelle Anwendung finden. Die Dimmschnittstelle ist vorzugsweise eine Schnittstelle, über die das Vorschaltgerät ein digitales Dimmsignal empfangen kann. Vorzugsweise wird der Dimmwert des Vorschaltgeräts in dem Vorschaltgerät abgespeichert, so dass es nach dem Aus- und Wiedereinschalten zunächst mit der zuletzt gewählten Betriebsstromeinstellung weiter betrieben wird, bis weitere Dimmsignale erhalten werden. Damit wird sichergestellt, dass ein an eine Lampenalterung angepasster Betriebsstromwert nicht bei jedem Einschalten des Solariums neu bestimmt werden muss.

Es kann alternativ vorgesehen werden, dass alle Vorschaltgeräte beim Einschalten des Solariums alle angeschlossenen Gasentladungslampen zunächst mit einem festgelegten oder ausgewählten Bruchteil der Gesamtleistung betreiben, wonach in einem kurzzeitigen Regelvorgang die Lampenströme auf die für den gewünschten UV-Lichtstrom für den erforderlichen Wert angepasst werden. Damit kann z.B. sichergestellt werden, dass ein zwischenzeitlich durchgeführter Lampenwechsel nicht zu einer Überschreitung der maximalen gewünschten UV-Bestrahlung führt.

Die zur Erfassung des UV-Lichtstroms vorgesehenen Sensoreinrichtungen sind vorzugsweise in der Nähe der Gasentladungslampen angeordnet. Im einfachsten Fall können jeder Gasentladungslampe eine Sensoreinrichtung und ein Vorschaltgerät zugeordnet sein. Die Sensoreinrichtung kann beispielsweise an der Lampenfassung oder an oder in dem der Gasentladungslampe zugeordneten Reflektor angeordnet sein. Die Sensoreinrichtung kann über eine geeignete Schnittstelle unmittelbar mit dem Vorschaltgerät verbunden sein. Die Steuereinrichtung zur Konstanthaltung bzw. Regelung des UV-Lampenstroms kann in diesem einfachen Sonderfall in das Vorschaltgerät eingebaut sein. In diesem Sonderfall umfasst das Beleuchtungssystem mehrere Vorschaltgeräte und auch mehrere gegebenenfalls in diese eingebaute Steuereinrichtungen.

Es ist auch möglich, mit jedem Vorschaltgerät zwei oder mehrere Gasentladungslampen zu betreiben. Auch in diesem Fall kann jedem Vorschaltgerät eine Sensoreinrichtung zugeordnet sein, die dann beispielsweise zwischen den beiden an das Vorschaltgerät angeschlossenen Gasentladungslampen angeordnet ist.

Es wird bevorzugt, die Vorschaltgeräte von nur einer Steuereinrichtung zu steuern, die mit den Vorschaltgeräten über entsprechende Schnittstellen verbunden und als gesondertes Gerät ausgebildet ist. Diese Steuereinrichtung kann in das Solarium integriert oder als gesondertes Gerät neben dem Solarium aufgebaut sein. Die Steuereinrichtung weist vorzugsweise eine Bedienschnittstelle auf. Diese kann Ein- und Ausgabemittel, Tasten, Wiedergabeeinrichtungen und dergleichen umfassen. Außerdem kann eine weitere Schnittstelle zur Verbindung mit einem zentralen Computer über ein lokales Netzwerk vorgesehen sein. Die Eingabemittel können dazu genutzt werden, beispielsweise UV-Lichtströme, UV-Bestrahlungsdosen, Expositionszeiten und dergleichen vorzugeben.

Die von der Steuereinrichtung gesteuerten Vorschaltgeräte können individuell oder in Gruppen gesteuert werden. Eine Gruppe kann jeweils nur ein Vorschaltgerät oder auch mehrere Vorschaltgeräte umfassen. Es ist auch möglich, dass alle vorhandenen Vorschaltgeräte in einer einzigen Gruppe zusammengefasst sind.

Jede Gruppe wird vorzugsweise gemäß der Signale gesteuert, die von einer oder mehreren Sensoreinrichtungen geliefert werden, die dieser Gruppe zugeordnet sind. Die Sensoreinrichtungen sind beispielsweise zwischen zwei Lampen einer Gruppe angeordnet und liefern somit ein Signal, das den UV-Lichtstrom kennzeichnet, der von dieser Gruppe erzeugt wird. Vorzugsweise hat die Sensoreinrichtung ein Empfindlichkeitsprofil, das dem der menschlichen Haut entspricht. Damit gelingt die Anpassung der erzeugten UV-Lichtströme an die Bedürfnisse der menschlichen Haut besonders gut.

Weitere Einzelheiten vorteilhafter Ausführungsformen der Erfindung sind Gegenstand der Beschreibung, der Zeichnung oder von Ansprüchen. Die Beschreibung beschränkt sich auf wesentliche Aspekte der Erfindung und sonstiger Gegebenheiten. Die Zeichnung offenbart weitere Details und ist ergänzend heranzuziehen. Es zeigen:
Figur 1 eine Bestrahlungseinrichtung in schematischer, perspektivischer Darstellung,
Figur 2 das Beleuchtungssystem der Bestrahlungseinrichtung nach Figur 1 in ausschnittsweiser, schematisierter Darstellung,
Figur 3 einen Ausschnitt aus dem Beleuchtungssystem nach Figur 2 in schematisierter Schnittdarstellung,
Figur 4 ein Blockbild des Beleuchtungssystems nach Figur 2 in schematisierter ausschnittsweiser Darstellung,
Figur 5 ein weiter vereinfachtes Blockbild eines Beleuchtungssystems der Bestrahlungseinrichtung in einer alternativen Ausführungsform,
Figur 6 UV-Intensitätskurven als Diagramm und
Figur 7 eine Empfindlichkeitskurve der Sensoreinrichtung als Diagramm.

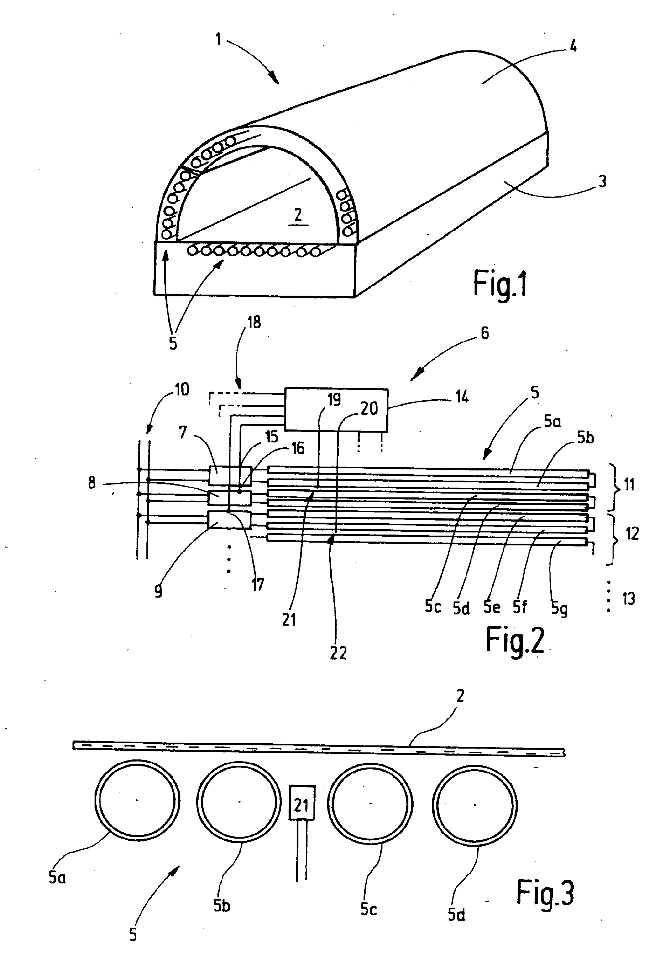

In Figur 1 ist eine Bestrahlungseinrichtung 1 veranschaulicht, die einen oben mit einer Liegefläche 2 versehenen unteren Abschnitt 3 und eine Haube 4 aufweist. Sowohl in dem unteren Abschnitt 3 wie auch in der Haube 4 sind Gasentladungslampen, beispielsweise in Form von Leuchtstofflampen 5 untergebracht. Die Liegefläche 2 ist die untere Begrenzung eines nach oben durch die Haube 4 abgeschlossenen Bestrahlungsraums, der von UV-Licht durchflutet wird. Die Liegefläche 2 kann durch eine Glasplatte oder ähnliches gebildet sein, die die Leuchtstofflampen 4 abdeckt. Die Leuchtstofflampen 5 sind zu dem Innenraum vorzugsweise ebenfalls von einer durchsichtigen Abdeckung getrennt.

Die Leuchtstofflampen 5 sind an eine Betriebseinrichtung 6 angeschlossen, wie sie ausschnittsweise aus Figur 2 hervorgeht. Zu der Betriebseinrichtung gehören vorzugsweise mehrere Vorschaltgeräte 7, 8, 9, deren Eingänge mit einem spannungsführenden Netz 10 verbunden sind. Dies kann ein ein- oder mehrphasiges öffentliches Versorgungsnetz mit 230 Volt oder auch 400 Volt sein. Die verschiedenen Vorschaltgeräte 7, 8, 9 können an einem Drehstromnetz an die gleiche Phase oder unterschiedlichen Phasen angeschlossen sein.

In Figur 2 sind mehrere Leuchtstofflampen 5a, 5b, 5c, 5d, 5e, 5f, 5g veranschaulicht. Jeweils zwei Leuchtstofflampen 5a, 5b; 5c, 5d; 5e, 5f usw. sind miteinander in Reihe geschaltet und über ein gemeinsames Vorschaltgerät 7, 8 oder 9 betrieben. Im vorliegenden Ausführungsbeispiel bilden die an die Vorschaltgeräte 7, 8 angeschlossenen vier Leuchtstofflampen 5a bis 5d eine Gruppe 11. Weitere Gruppen 12, 13 usw. setzen die Reihe der Leuchtstofflampen 5 fort.

Zu der Betriebseinrichtung 6 gehört eine Steuereinrichtung 14, die dazu dient, die Vorschaltgeräte 7, 8, 9 usw. zu steuern. Dazu weisen die Vorschaltgeräte 7, 8, 9 jeweils Eingangsschnittstellen 15, 16, 17 auf, die über geeignete Leitungen 18 oder andere Signalübertragungsstrecken mit der Steuereinrichtung 14 verbunden sind. Die Eingangsschnittsstellen 15, 16, 17 sind vorzugsweise digitale Schnittstellen, über die der von dem jeweiligen Vorschaltgerät 7, 8, 9 gelieferte Lampenstrom festzulegen ist.

Die Steuereinrichtung 14 weist außerdem mehrere Eingänge 19, 20 auf, die mit Sensoreinrichtungen 21, 22 verbunden sind. Diese Sensoreinrichtungen 21, 22 sind vorzugsweise in unmittelbarer Nachbarschaft zu den Leuchtstofflampen 5 angeordnet. Außerdem ist vorzugsweise für jede Gruppe 11, 12 usw. jeweils eine Sensoreinrichtung 21, 22 usw. zugeordnet. Alternativ können jeder Gruppe 11, 12 auch mehrere Sensoreinrichtungen individuell zugeordnet sein. Die Sensoreinrichtungen 21, 22 dienen dazu, den von den Leuchtstofflampen der Gruppen 11, 12 ausgesandten Lichtstrom zu erfassen und entsprechende Signale an die Steuereinrichtung 14 zu liefern. Z.B. umfasst die Gruppe 11 zwei Lampenzweige mit jeweils zwei in Reihe geschalteten Leuchtstofflampen. Die Sensoreinrichtung 21 ist vorzugsweise zwischen zwei Leuchtstofflampen 5b, 5c angeordnet, die zu unterschiedlichen Lampenzweigen ein und derselben Gruppe 11 gehören. Die Sensoreinrichtung 21 erfasst somit das UV-Licht beider Lampenzweige.

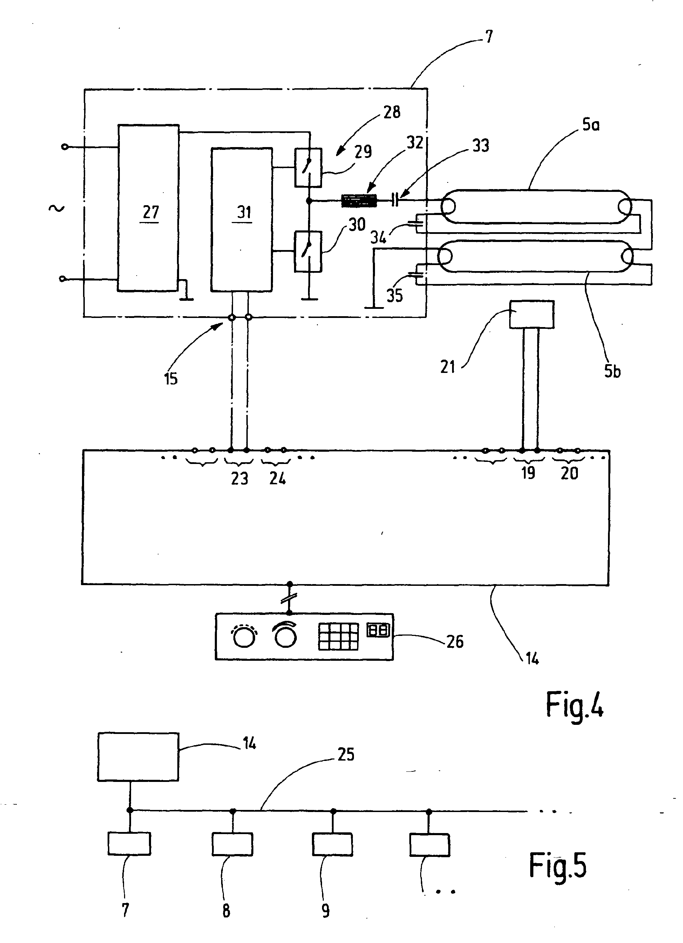

Figur 4 veranschaulicht das Vorschaltgerät 7 mit den angeschlossenen Leuchtstofflampen 5a, 5b sowie die Steuereinrichtung 14 und die Sensoreinrichtung 21. Die Sensoreinrichtung 21 ist an den Eingang 19 angeschlossen. Ein Steuerausgang 23 der Steuereinrichtung 14 ist mit der Eingangsschnittstelle 15 verbunden. Die Steuereinrichtung 14 kann für alle Sensoreinrichtungen 21, 22 usw. individuelle Eingänge 19, 20 und auch für die Vorschaltgeräte 7, 8 usw. individuelle Ausgänge 23, 24 aufweisen. Es ist jedoch ebenso gut möglich, verschiedene Vorschaltgeräte 7, 8, 9 usw. über einen Bus 25 mit der Steuereinrichtung 14 zu verbinden, wie Figur 5 zeigt. Gleiches gilt für die Sensoreinrichtungen 21, 22.

Die Steuereinrichtung 14 kann eine Bedienschnittstelle aufweisen oder mit einem Bediengerät 26 verbunden sein. Dieses kann Ein- und Ausgabemittel wie Knöpfe, Tasten, Anzeigebildschirme, alphanumerische Anzeigen, Kontrolllampen und dergleichen aufweisen. Außerdem kann die Steuereinrichtung 14 mit einer nicht weiter veranschaulichten Schnittstelle versehen sein, um an ein Computernetzwerk angeschlossen zu werden. Es kann eine Internet-Schnittstelle, eine LAN-Schnittstelle für ein leitungsgebundenes oder leitungsloses Netz vorgesehen sein.

Das Vorschaltgerät 7 enthält eine Gleichrichter- und Gleichspannungserzeugungsbaugruppe 27, die aus einer Netzwechselspannung von beispielsweise 230 Volt eine Gleichspannungszwischenkreisspannung von z.B. 400 Volt erzeugt. Diese speist einen Wechselrichter 28, der z.B. zwei wechselweise öffnende und schließende elektronische Schalter 29, 30 z.B. in Form von IGBTs, FETTs oder dergleichen enthält. Die beiden elektronischen Schalter 29 werden von einer Ansteuerschaltung 31 gesteuert, die die Schalter 29, 30 mit Schaltimpulsen versorgt, um sie mit festgelegter Frequenz zu öffnen und zu schließen.

Der Wechselrichter 28 ist über eine strombegrenzende Drossel 32 mit dem Lampenzweig verbunden. Die Drossel 32 weist eine induktive Impedanz auf. Mit ihr kann ein Koppelkondensator 33 in Reihe geschaltet sein, um an dem Wechselrichterausgang 28 anstehende Gleichspannungsanteile von den Leuchtstofflampen 5a, 5b fernzuhalten. Letztere sind in Reihe geschaltet, wobei ihnen Zündkondensatoren 34, 35 parallel geschaltet sein können. Diese dienen zum Vorheizen der Heizfäden der Leuchtstofflampen 5a, 5b. Zum Vorheizen wird der Wechselrichter 28 mit einer Frequenz betrieben, die so hoch ist, dass ein wesentlicher Strom durch die Zündkondensatoren 34, 35 und die mit diesen in Reihe geschalteten Heizfäden fließt. Zum zeitweiligen oder dauernden Heizen der Lampenelektroden sind auch andere Heizschaltungen möglich. Die in Figur 4 dargestellte Schaltung stellt nur ein nicht beschränkendes Beispiel dar.

Über die Ansteuerschaltung 31 kann sowohl die Schaltfrequenz des Wechselrichters 28 als auch die Einschaltdauern der einzelnen Schalter 29, 30 und damit den durch die Leuchtstofflampen 5a, 5b fließenden Strom bestimmen. Die Ansteuerschaltung ist mit der Eingangsschnittstelle 15 verbunden und erhält somit von der Steuereinrichtung 14 entsprechende Vorgaben.

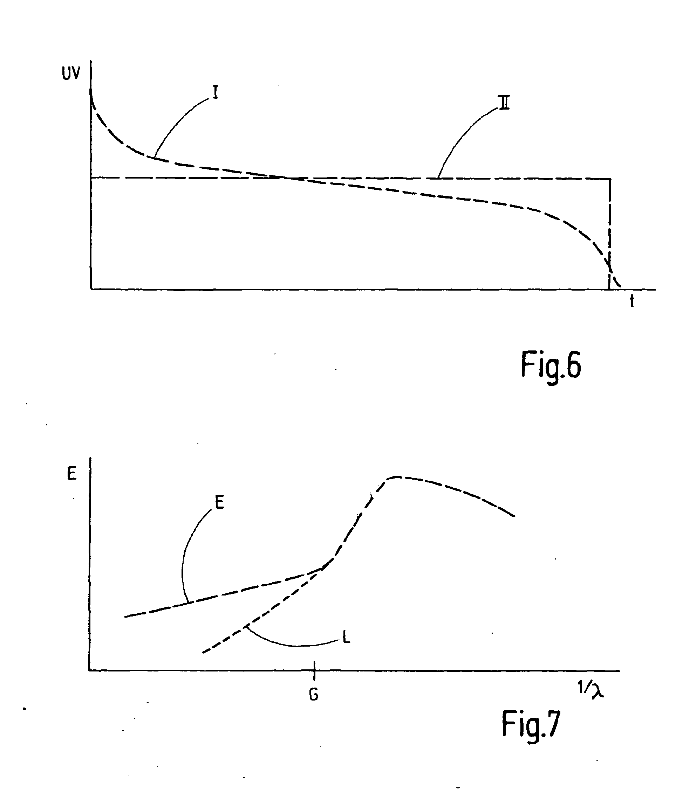

Die Sensoreinrichtung 21 ist in unmittelbarer Nachbarschaft der Leuchtstofflampen 5a, 5b angeordnet. Vorzugsweise reagiert die Sensoreinrichtung vorwiegend auf UV-Licht. Sie gibt somit an den Eingang 19 ein Signal ab, das die empfangene UV-Strahlung kennzeichnet. Vorzugsweise weist die Sensoreinrichtung 21 dabei eine Empfindlichkeitskurve auf, der der menschlichen Haut entspricht. Eine solche Empfindlichkeitskurve ist beispielhaft in Figur 7 dargestellt. An der Vertikalachse ist der von der jeweiligen Wellenlänge hervorgerufene Effekt aufgetragen während die Horizontalachse durch die Wellenlänge bestimmt ist. Die beispielhaft veranschaulichte Kurve zeigt einen mit abnehmender Wellenlänge zunehmenden Effekt für den hier relevanten Bereich des elektromagnetischen Spektrums. Vorzugsweise ist die Signalstärke an dem Ausgang der Sensoreinrichtung 21 in gleichem Maße von der Wellenlänge wie der Effekt der Strahlung auf die menschliche Haut. Die Sensoreinrichtung 21 kann an ihrem Ausgang Digitalsignale liefern, die somit diesen Effekt kennzeichnen.

Die Steuereinrichtung 14 kann die Sensorsignale auf verschiedene, gegebenenfalls auch durch das Bediengerät 26 konfigurierbare Weise bearbeiten. Beispielsweise kann es möglich sein, eine maximale UV-Strahlungsleistung einzustellen, eine maximale UV-Strahlungsdosis einzustellen, die Strahlungsleistung oder die Strahlungsdosis in Abhängigkeit vom Hauttyp einzustellen, vorgewählte Bestrahlungsprofile einzustellen, beispielsweise mit einer gewissen Zeitspanne schwächerer UV-Intensität, einer Zeitspanne höherer UV-Intensität und einer anderen Zeitspanne wieder verminderter UV-Intensität oder dergleichen.

Die insoweit beschriebene Betriebseinrichtung 6 arbeitet wie folgt:

In Betrieb überwacht die Steuereinrichtung 14 mittels der Sensoreinrichtungen 21, 22 die von den verschiedenen Lampengruppen 11, 12, 13 usw. abgegebene UV-Leistung. Die die einzelnen Lampengruppen 11, 12, 13 speisenden Vorschaltgeräte 7, 8, 9 werden danach jeweils so reguliert, dass möglichst alle Lampengruppen die gewünschte UV-Strahlungsleistung bzw. UV-Leistungsdichte erzeugen. Dies kann unabhängig vom Alterungsgrad der Leuchtstofflampen erfolgen. Z.B. können Lampengruppen, die frisch bestückt worden sind, mit geringerem Betriebsstrom betrieben werden, während schon fortgeschritten gealterte Leuchtstofflampen einen entsprechend höheren Betriebsstrom erhalten, um noch die gewünschte UV-Strahlungsleistung zu erbringen.

Figur 6 veranschaulicht mit einer ersten Kurve I den Verlauf der UV-Leistung über der Lebenszeit einer Leuchtstofflampe qualitativ. Es ist zu erkennen, dass neue Leuchtstofflampen eine sehr hohe UV-Ausbeute erbringen, die relativ schnell auf ein niedrigeres Maß abfällt. Gegen Ende der Lampenlebensdauer fällt die UV-Strahlung dann weiter stark ab. Mit dem erfindungsgemäßen System ist es möglich, die UV-Leistung konstant zu halten, wie die Kurve II zeigt. Zu Beginn der Lebensdauer der Leuchtstofflampe wird deren Betriebsstrom stark reduziert, so dass sie mit verminderter UV-Leistung arbeitet. Nach und nach wird der Betriebsstrom so lange erhöht, dass trotz fortschreitender Alterung der Leuchtstofflampe deren UV-Leistung aufrecht erhalten wird. Ist das Lebensdauerende erreicht, kann die Lampe stillgesetzt werden. Dadurch wird es zusätzlich zur Konstanthaltung der UV-Leistung möglich, die Lebensdauer der Leuchtstofflampen zu verlängern. Die Alterung verzögert sich und selbst gegen Lebensdauerende wird noch die erforderliche UV-Leistung erbracht.

Die Einstellung des Lampenstroms kann fortwährend im Rahmen einer geschlossenen Regelschleife erfolgen. Es ist aber auch möglich, dass das Steuergerät 14 einmal eingestellte Lampenströme jeweils für eine gewisse Zeit, beispielsweise mehrere Minuten beibehält, um lediglich von Zeit zu Zeit die Sensoreinrichtungen 21, 22 usw. abzufragen und, falls erforderlich, den Lampenstrom nachzuregulieren. Dies ist insbesondere dann sinnvoll, wenn die Vorschaltgeräte 7, 8, 9 und die Sensoren 21, 22 usw. beispielsweise über einen Bus miteinander verbunden sind, dessen Datenrate beschränkt ist.

Bei der erfindungsgemäßen Bestrahlungseinrichtung werden mittels eines oder mehreren Sensoren 21, 22 (z.B. UV-Fotodiode, Erythemsensor) die aktuelle UV-Lichtleistung oder die Strahlungsdosis bestimmt, die auf den zu bestrahlenden Körper wirkt. Der Sensor 21, 22 kann eine auf die menschliche Haut abgestimmte Empfindlichkeitskurve aufweisen, wie sie in Figur 7 dargestellt ist. Die Empfindlichkeitskurve der Haut ist in Figur 7 mit "E" bezeichnet. Wie ersichtlich nimmt mit abnehmender Wellenlänge die Wirkung der UV-Strahlung auf die Haut zu.

Es kann sich bei der Fotodiode um eine SiC-Diode handeln. Diese kann zusätzlich mit einem UV-Filter versehen sein, das in Verbindung mit der Fotodiode die in Figur veranschaulichte Empfindlichkeitskurve L ergibt. Der Sensor kann mit einem Signalverstärker zu einem aktiven Sensor zusammengefasst sein, um ein gutes Ausgangssignal zu erzeugen. Bei größeren Wellenlängen müssen die Empfindlichkeitskurven L und E nicht unbedingt übereinstimmen. Ab einer Grenze G, d.h. bei niedrigeren Wellenlängen stimmen die Empfindlichkeitskurven L und E jedoch zumindest in guter Näherung überein. Mit abnehmender Wellenlänge (zunehmender Frequenz) steigt das Ausgangssignal ab der Grenze G zunächst steil an und fällt dann flach wieder ab. Das von dem Sensor erzeugten Signal verhält sich so wie die Empfindlichkeit der Haut in Bezug auf die kurzwelligeren Anteile des Lichts. Somit haben der Sensor und die Haut im interessierenden Wellenlängenbereich die gleich relative spektrale Empfindlichkeit. Somit können der Sensor und das Steuergerät die physiologische Strahlungsdosis ermitteln und begrenzen.

Beispielsweise kann für jedes Teilsegment eines Bestrahlungsgeräts (Liegefläche, Seitenwand, Haube) jeweils ein Sensor vorgesehen sein. Nach Ermittlung der Strahlungsparameter werden diese an eine Steuereinrichtung (Lichtsteuereinheit) weitergegeben und in dieser ausgewertet. Nach abgeschlossener Auswertung gibt die Lichtsteuereinheit ein Steuersignal an die elektronischen Vorschaltgeräte 7, 8, 9 aus, die bei Bedarf den Lampenstrom (oder evtl. auch andere Parameter, die die Lichtleistung bzw. Strahlungsdosis beeinflussen) verringern oder erhöhen. Gegebenenfalls können Betriebsdaten des Vorschaltgeräts z.B. zur Realisierung eines Überlastschutzes ebenfalls an das Steuergerät gesendet und in die Steuerstrategien miteinbezogen werden.

Es kann sichergestellt werden, dass die gesetzlich maximal vorgegebene Strahlungsdosis nie überschritten wird, selbst wenn versehentlich Lampenfehlbestückungen vorgenommen werden. Auch kann eine erhebliche Verlängerung der Lampenstandzeiten erreicht werden. Die Bestrahlungseinrichtung 1 ist Dank der Betriebseinrichtung 6 sicherer und verlässlicher.

### Bezugszeichen

- 1: Bestrahlungseinrichtung
- 2: Liegefläche
- 3: Unterteil
- 4, 5: Leuchtstofflampen/ Gasentladungslampen
- 6: Betriebseinrichtung
- 7, 8, 9: Vorschaltgerät
- 10: Netz
- 11, 12, 13: Gruppen
- 14: Steuereinrichtung
- 15, 16, 17: Eingangsschnittstellen
- 18: Leitungen
- 19, 20: Eingänge
- 21, 22: Sensoreinrichtungen
- 23, 24: Ausgänge
- 25: Bus
- 26: Bediengerät
- 27: Gleichrichter und Gleichspannungserzeugungsbaugruppe
- 28: Wechselrichter
- 29, 30: Schalter
- 31: Ansteuerschaltung
- 32: Drossel
- 33: Koppelkondensator
- 34, 35: Zündkondensatoren

## Patentansprüche

1. Betriebseinrichtung (6) für zumindest eine Gasentladungslampe (5a) einer Bestrahlungseinrichtung (1),
mit mindestens einem Vorschaltgerät (7) zum Betrieb der mindestens einem Gasentladungslampe (5a) und
mit mindestens einer Sensoreinrichtung (21) zur Erfassung von UV-Strahlung, die von der zumindest einen Gasentladungslampe (5a) ausgestrahlt wird, und
mit einer Steuereinrichtung (14), die mit der Sensoreinrichtung (21) und dem Vorschaltgerät (7) verbunden ist, um das Vorschaltgerät (7) anhand zumindest eines Signals zu steuern, das die Sensoreinrichtung (21) liefert,
**dadurch gekennzeichnet, dass** die Sensoreinrichtung (21) eine Abhängigkeit (L) der Ausgangssignalstärke von der Lichtwellenlänge hat, die zumindest für Lichtwellenlängen kleiner als eine Gren ze (G) mit dem Empfindlichkeitsprofil (E) der menschlichen Haut in guter Näherung übereinstimmt.

2. Betriebseinrichtung nach Anspruch 1, **dadurch gekennzeichnet, dass** das Vorschaltgerät (7) eine Einrichtung (31) zur Beeinflussung eines durch die Gasentladungslampe (5a) fließenden Betriebsstroms aufweist.

3. Betriebseinrichtung nach Anspruch 1, **dadurch gekennzeichnet, dass** das Vorschaltgerät (7) eine Wechselrichterschaltung (28) aufweist, die von einer Ansteuerschaltung (31) angesteuert wird, um eine Wechselspannung mit gewünschter Frequenz zu erzeugen.

4. Betriebseinrichtung nach Anspruch 1, **dadurch gekennzeichnet, dass** zwischen der Wechselrichterschaltung (28) und der Gasentladungslampe (5a) wenigstens ein Element (32) mit frequenzabhängiger Impedanz angeordnet ist, um einen durch die Gasentladungslampe (5a) fließenden Betriebsstrom in Abhängigkeit von der Frequenz der Wechselrichterspannung steuern zu können.

5. Betriebseinrichtung nach Anspruch 1, **dadurch gekennzeichnet, dass** die Sensoreinrichtung (21) ein Signal erzeugt, das die Intensität der aufgenommenen UV-Strahlung kennzeichnet, wobei dieses Signal an die Steuereinrichtung (14) geliefert wird.

6. Betriebseinrichtung nach Anspruch 1, **dadurch gekennzeichnet, dass** das Vorschaltgerät (7), die Steuereinrichtung (14) und ein oder mehrere Sensoreinrichtungen (21, 22) als gesonderte Geräte ausgebildet sind, die zur Kommunikation miteinander vorbereitete Schnittstellen (15, 19, 23) aufweisen.

7. Betriebseinrichtung nach Anspruch 1, **dadurch gekennzeichnet, dass** die Steuereinrichtung (14) die von den Gasentladungslampen (5a) erzeugte UV-Leistung konstant hält.

8. Betriebseinrichtung nach Anspruch 1, **dadurch gekennzeichnet, dass** die Steuereinrichtung (14) die von den Gasentladungslampen (5a) ausgestrahlte UV-Leistung einem Zeitprofil folgend variiert.

9. Betriebseinrichtung nach Anspruch 1, **dadurch gekennzeichnet, dass** die Steuereinrichtung (14) die von den Gasentladungslampen (5a) ausgestrahlte UV-Dosis einem Vorgabewert entsprechend begrenzt.

10. Betriebseinrichtung nach Anspruch 1, **dadurch gekennzeichnet, dass** an jedes Vorschaltgerät (7) mindestens ein Lampenzweig angeschlossen ist, der wenigstens eine Gasentladungslampe (5a) enthält.

11. Betriebseinrichtung nach Anspruch 1, **dadurch gekennzeichnet, dass** an jedes Vorschaltgerät (7) mindestens ein Lampenzweig angeschlossen ist, der wenigstens zwei Gasentladungslampen (5a, 5b) enthält.

12. Betriebseinrichtung nach Anspruch 1, **dadurch gekennzeichnet, dass** zwei Gasentladungslampen (5a, 5b) eines Lampenzweigs miteinander in Reihe geschaltet sind.

13. Betriebseinrichtung nach Anspruch 1, **dadurch gekennzeichnet, dass** die Gasentladungslampen (5a, 5b) eines Lampenzweigs nebeneinander angeordnet und gegensinnig stromdurchflossen sind.

14. Betriebseinrichtung nach Anspruch 1, **dadurch gekennzeichnet, dass** die Vorschaltgeräte (7, 8, 9) in Gruppen (11, 12) eingeteilt sind, wobei jeder Gruppe (11, 12) mindestens eine Sensoreinrichtung (21, 22) zugeordnet ist, um die jeweilige Gruppe (11, 12) zu steuern.

15. Betriebseinrichtung nach Anspruch 1, **dadurch gekennzeichnet, dass** jede Sensoreinrichtung (21, 22) mindestens einen Sensor aufweist.

16. Betriebseinrichtung nach Anspruch 14 und 15, **dadurch gekennzeichnet, dass** der Sensor unmittelbar dem Licht ausgesetzt ist, das von zumindest einer Gasentladungslampe (5b, 5c) der Gruppe (11) ausgesandt wird.

17. Bestrahlungseinrichtung mit einem Gehäuse, in dem eine Liegefläche (2) für eine Person und mehrere Gasentladungslampen (4, 5) angeordnet sind, mit einer Betriebseinrichtung (6) nach einem der vorstehenden Ansprüche, wobei die Gasentladungslampen (4, 5) mit der Betriebseinrichtung (6) verbunden sind.

## Claims

1. Operating device (6) for at least one gas-discharge lamp (5a) of an irradiation system (1),
comprising at least one power supply unit (7) for operating said at least one gas-discharge lamp (5a) and
at least one sensor device (21) for detecting ultraviolet radiation, which is radiated by said at least one gas-discharge lamp (5a), and a control device (14), which is connected with said sensor device (21) and said power supply unit (7) in order to control said power supply unit (7) on the basis of at least one signal, which is delivered by said sensor device (21),
**characterized in that** said sensor device (21) has a dependence (L) of the output signal strength on the light wave length, which at least for light wave lengths of less than one limit (G) corresponds in good approximation with the sensitivity profile (E) of the human skin.

2. Operating device according to claim 1, **characterized in that** said power supply unit (7) includes a trigger circuit (31) for influencing an operational current flowing through said gas-discharge lamp (5a).

3. Operating device according to claim 1, **characterized in that** said power supply unit (7) includes an inverter circuit (28), which is controlled by a trigger circuit (31), in order to generate an alternating voltage with desired frequency.

4. Operating device according to claim 1, **characterized in that** at least one element (32) with frequency-dependent impedance is located between said inverter circuit (28) and said gas-discharge lamp (5a), in order to be able to control an operational current flowing through said gas-discharge lamp (5a) as a function of the frequency of the inverter voltage.

5. Operating device according to claim 1, **characterized in that** said sensor device (21) generates a signal, which indicates the intensity of the absorbed ultraviolet radiation, wherein this signal is delivered to said control device (14).

6. Operating device according to claim 1, **characterized in that** said power supply unit (7), said control device (14) and one or several devices (21, 22) are formed as separate devices, which have interfaces (15, 19, 23) prepared for communication with one another.

7. Operating device according to claim 1, **characterized in that** said control device (14) keeps the ultraviolet output produced by said gas-discharge lamps (5a) constant.

8. Operating device according to claim 1, **characterized in that** said control device (14) varies the ultraviolet output radiated from said gas-discharge lamps (5a) following a time profile.

9. Operating device according to claim 1, **characterized in that** said control device (14) restricts the ultraviolet dose radiated from said gas-discharge lamps (5a) according to a predetermined value.

10. Operating device according to claim 1, **characterized in that** at least one lamp branch is connected to each power supply unit (7), which contains at least one gas-discharge lamp (5a).

11. Operating device according to claim 1, **characterized in that** at least one lamp branch is connected to each power supply unit (7), which contains at least two gas-discharge lamps (5a, 5b).

12. Operating device according to claim 1, **characterized in that** two gas-discharge lamps (5a, 5b) of a lamp branch are switched in series with one another.

13. Operating device according to claim 1, **characterized in that** said gas-discharge lamps (5a, 5b) of a lamp branch are located next to each other and are energized in counter-directions.

14. Operating device according to claim 1, **characterized in that** said power supply units (7, 8, 9) are divided into groups (11, 12), wherein at least one sensor device (21, 22) is associated with each group (11, 12), in order to control the respective group (11, 12).

15. Operating device according to claim 1, **characterized in that** each sensor device (21, 22) has at least one sensor.

16. Operating device according to claim 14 and 15, **characterized in that** said sensor is directly exposed to the light, which is emitted by at least one gas-discharge lamp (5b, 5c) of the group (11).

17. Irradiation system with a housing, in which a lying surface (2) for one person and several gas-discharge lamps (4, 5) are located, comprising an operating device (6) according to any one of the above claims, wherein said gas-discharge lamps (4, 5) are connected with said operating device (6).

## Revendications

1. Dispositif d'exploitation (6) pour au moins une lampe à décharge gazeuse (5a) d'un système de rayonnement (1),
comprenant au moins un ballast (7) pour faire fonctionner la lampe à décharge gazeuse (5a), au nombre d'au moins une, et
comprenant au moins un dispositif capteur (21) destiné à détecter un rayonnement UV qui est émis par la lampe à décharge gazeuse (5a), au nombre d'au moins une, et
comprenant un dispositif de commande (14) qui est relié au dispositif capteur (21) et au ballast (7) pour commander le ballast (7) à l'aide d'au moins un signal qui est fourni par le dispositif capteur (21),
**caractérisé en ce que** le dispositif capteur (21) présente une fonction (L) de l'intensité de signal de sortie vis-à-vis de la longueur d'onde lumineuse, qui coïncide avec une bonne approximation avec le profil de sensibilité (E) de la peau humaine, au moins pour des longueurs d'ondes lumineuses inférieures à une limite (G).

2. Dispositif d'exploitation selon la revendication 1, **caractérisé en ce que** le ballast (7) présente un dispositif (31) pour influencer un courant de service parcourant la lampe à décharge gazeuse (5a).

3. Dispositif d'exploitation selon la revendication 1, **caractérisé en ce que** le ballast (7) présente un circuit onduleur (28) qui est activé par un circuit d'activation (31) pour générer une tension alternative de fréquence souhaitée.

4. Dispositif d'exploitation selon la revendication 1, **caractérisé en ce qu'**il est prévu entre le circuit onduleur (28) et la lampe à décharge gazeuse (5a), au moins un élément (32) à impédance variant avec la fréquence, afin de pouvoir contrôler un courant de service parcourant la lampe à décharge gazeuse (5a), en fonction de la fréquence de la tension de l'onduleur.

5. Dispositif d'exploitation selon la revendication 1, **caractérisé en ce que** le dispositif capteur (21) génère un signal qui caractérise l'intensité du rayonnement UV absorbé, ledit signal étant fourni au dispositif de commande (14).

6. Dispositif d'exploitation selon la revendication 1, **caractérisé en ce que** le ballast (7), le dispositif de commande (14) et un ou plusieurs dispositifs capteurs (21, 22) sont réalisés sous forme d'appareils distincts qui présentent des interfaces (15, 19, 23) préparées pour communiquer les unes avec les autres.

7. Dispositif d'exploitation selon la revendication 1, **caractérisé en ce que** le dispositif de commande (14) maintient constante la puissance UV produite par les lampes à décharge gazeuse (5a).

8. Dispositif d'exploitation selon la revendication 1, **caractérisé en ce que** le dispositif de commande (14) fait varier en fonction d'un profil chronologique la puissance UV émise par les lampes à décharge gazeuse (5a).

9. Dispositif d'exploitation selon la revendication 1, **caractérisé en ce que** le dispositif de commande (14) limite en fonction d'une valeur prédéfinie la dose UV émise par les lampes à décharge gazeuse (5a).

10. Dispositif d'exploitation selon la revendication 1, **caractérisé en ce qu'**est connectée à chaque ballast (7), au moins une branche de lampe qui comporte au moins une lampe à décharge gazeuse (5a).

11. Dispositif d'exploitation selon la revendication 1, **caractérisé en ce qu'**est connectée à chaque ballast (7), au moins une branche de lampe qui comporte au moins deux lampes à décharge gazeuse (5a, 5b).

12. Dispositif d'exploitation selon la revendication 1, **caractérisé en ce que** deux lampes à décharge gazeuse (5a, 5b) d'une branche de lampe sont montées en série l'une avec l'autre.

13. Dispositif d'exploitation selon la revendication 1, **caractérisé en ce que** les lampes à décharge gazeuse (5a, 5b) d'une branche de lampe sont disposées l'une à côté de l'autre et sont traversées par le courant dans des sens opposés.

14. Dispositif d'exploitation selon la revendication 1, **caractérisé en ce que** les ballasts (7, 8, 9) sont divisés en groupes (11, 12), sachant qu'à chaque groupe (11, 12) est associé au moins un dispositif capteur (21, 22) pour commander le groupe (11,12) respectif.

15. Dispositif d'exploitation selon la revendication 1, **caractérisé en ce que** chaque dispositif capteur (21, 22) présente au moins un capteur.

16. Dispositif d'exploitation selon les revendications 14 et 15, **caractérisé en ce que** le capteur est directement exposé à la lumière qui est émise par au moins une lampe à décharge gazeuse (5b, 5c) du groupe (11).

17. Système de rayonnement comprenant un boîtier dans lequel sont installées une surface pour s'allonger (2) pour une personne, ainsi que plusieurs lampes à décharge gazeuse (4, 5), comprenant un dispositif d'exploitation (6) selon l'une des revendications précédentes, les lampes à décharge gazeuse (4, 5) étant reliées au dispositif d'exploitation (6).
